# EUROPEAN PATENT APPLICATION

(11) **EP 4 245 211 A1**
(43) Date of publication of application: **20.09.2023**
(21) Application number: 20961706.7
(22) Date of filing: 20.11.2020
(51) Int. Cl.: A61B 5/00, A61B 1/00, A61B 1/07, A61M 25/01

(54) **OPTICAL COUPLING DEVICE**

(30) Priority: 11.11.2020 KR 20200150428
(71) Applicant: Dotter Inc., Incheon 22004 (KR)
(72) Inventor: KIM, Hungil, Incheon 22001 (KR); CHOI, Sogi, Seoul 06627 (KR); ANDREW, Burns, Burke, Virginia 22015 (US); YOSUF, Ahmed, Québec H9X 1B8 (CA)
(74) Representative: Nordmeyer, Philipp Werner
(86) International application number: PCT/KR2020/016482
(87) International publication number: WO 2022/102836

(57) **Abstract**

According to some embodiments of the present disclosure, an optical coupling apparatus is disclosed. The optical coupling apparatus is an apparatus for transmitting an optical signal from a static signal path to a rotating signal path, and may include: a fixing portion comprising a first optical path having an optical termination directed in a downstream direction; a rotating portion comprising a second optical path and having a source of a rotation motion, the second optical path including a first end portion directed in a direction where the optical termination is located and a second termination directed in a direction where a downstream rotation optical path is located; and a connecting portion providing a connection between the rotating portion and the downstream rotation optical path.

## Description

### [Technical Field]

The present disclosure relates to an optical coupling apparatus, and more particularly, to an optical coupling apparatus having a rotation coupling structure.

### [Background]

In the human body, it is known that the coronary artery can play a significant role in supplying oxygen and nutrients to the heart muscle. However, in modern society, the population with many coronary artery diseases is increasing due to irregular eating habits or stress.

In order to diagnose such coronary artery diseases, there are various imaging technologies for imaging internal structures of blood vessels. For example, there are angiography, intravascular ultrasound (IVUS), and optical coherence tomography (OCT) technologies.

The optical coherence tomography technology is in the spotlight as a next-generation blood vessel imaging technology by providing sensitivity as well as excellent image resolution. The optical coherence tomography technology may be performed by emitting light through a catheter.

Specifically, a catheter may be inserted into a patient's blood vessel. When imaging an inside of a blood vessel while rotating the inserted catheter at a predetermined rotating speed and simultaneously performing pullback, the inside of the blood vessel may be imaged.

In this case, the catheter only performs a function of emitting light transmitted from an optical coherence tomography apparatus, and it may be difficult for the catheter itself to generate and emit light. Therefore, in the related art, the fixed optical coherence tomography apparatus and the catheter are connected using a rotary joint or the like. However, according to the related art, various wires are provided around a motor provided for rotating the catheter, so that various problems such as vibration and noise may occur.

Therefore, there is a need to develop an optical coupling apparatus capable of providing rotation coupling without generating vibration and noise.

### [Summary]

The present disclosure has been made in view of the circumstances described above, and is intended to provide an optical coupling apparatus having a rotation coupling structure.

The technical problem addressed by the present disclosure is not limited to the problem described above, and other problems not described will be apparently understood by one skilled in the art from the following description.

According to an exemplary embodiment of the present disclosure for solving the problem described above, an optical coupling apparatus is disclosed. The optical coupling apparatus is an apparatus for transmitting an optical signal from a static signal path to a rotating signal path including: a fixing portion including a first optical path having an optical termination directed in a downstream direction; a rotating portion including a second optical path and having a source of a rotation motion, the second optical path including a first end portion directed in a direction where the optical termination is located and a second termination directed in a direction where a downstream rotation optical path is located; and a connecting portion providing a connection between the rotating portion and the downstream rotation optical path.

In addition, the connecting portion may include a pair of counterweights that increases clamping force of the connection between the rotating portion and the downstream rotation optical path as a rotation speed of the rotating portion increases.

In addition, the pair of counterweights may include at least one notch formed along a circumference and notches of the pair of counterweights may be provided to face each other.

In addition, the pair of counterweights may be engaged with each other so that central axes of counterweights are not matched and rotation axes of counterweights are matched.

In addition, the connecting portion may include an elastic member that maintains the connection between the rotating portion and the downstream rotation optical path, when the rotating portion does not rotate.

In addition, the second optical path may be an optical fiber configured to rotate with the rotation portion together when the rotation portion rotates.

In addition, the source of the rotation motion may be a motor, and the optical fiber may rotate while being coupled to a rotor of the motor.

In addition, the rotor may include a hollow shaft to which the optical fiber is inserted.

In addition, the downstream rotation optical path may be provided to be attachable to the connecting portion or to be detachable from the connecting portion.

In addition, the connecting portion may include a fiber connector that fixes the downstream rotation optical path, in which the fiber connector may include: a slot provided on an outer circumferential surface of the fiber connector; a pin which is movable while being inserted to the slot; and a pair of fingers which separates the downstream rotation optical path or fixes the downstream rotation optical path according to a position of the slot in which the pin is located.

Further, a method for transmitting an optical signal from a static signal path to a rotating signal path may be provided. The method may include: coupling an apparatus and a downstream rotation optical path; receiving an optical signal from a first optical path having an optical termination directed in a downstream direction, the first optical path being included in a fixing portion of the apparatus; and rotating a rotating portion of the apparatus and the downstream rotation optical path together through a connecting portion of the apparatus, the connecting portion providing a connection between the rotating portion and the downstream rotation optical path.

The technical solutions obtainable in the present disclosure are not limited to the solutions described above, and other solutions not described will be apparently understood by one skilled in the art from the following description.

According to some embodiments of the present disclosure, it is possible to provide the optical coupling apparatus that does not generate vibration, noise and the like.

The effects obtainable in the present disclosure are not limited to the effects described above, and other effects not described will be apparently understood by one skilled in the art from the following description.

### [Description of Drawings]

Various aspects will now be described with reference to the drawings. Here, similar reference numbers will be used to collectively refer to similar constitutional elements. In the following embodiments, for the sake of description, a plurality of specific details will be presented to provide overall understanding of one or more aspects. However, it will be apparent that such aspect(s) may be embodied without the specific details. In other examples, known structures and apparatuses are shown in the form of a block diagram to facilitate description of one or more aspects.
FIG. 1 is a front view for illustrating an example of an optical coupling apparatus according to some embodiments of the present disclosure.
FIG. 2 is a cross-sectional view taken along a line A-A' of FIG. 1 for illustrating the example of the optical coupling apparatus according to some embodiments of the present disclosure.
FIG. 3 is a detailed view of FIG. 2 for illustrating the example of the optical coupling apparatus according to some embodiments of the present disclosure.
FIG. 4 is a view for illustrating an example of a first aspect according to some embodiments of the present disclosure.
FIG. 5 is a detailed view of FIG. 4 for illustrating the example of the first aspect according to some embodiments of the present disclosure.
FIG. 6 is a view for illustrating an example of a second aspect according to some embodiments of the present disclosure.
FIG. 7 is a detailed view of FIG. 6 for illustrating the example of the second aspect according to some embodiments of the present disclosure.
FIG. 8 is a front view for illustrating an example of a fiber connector according to some embodiments of the present disclosure.
FIG. 9 is a plan view for illustrating the example of the fiber connector according to some embodiments of the present disclosure.
FIG. 10 is a view for illustrating an example of a pair of counterweights according to some embodiments of the present disclosure.
FIG. 11 is an exploded perspective view for illustrating an example of an optical coupling apparatus according to some embodiments of the present disclosure.

### [Detailed Description]

Various embodiments and/or aspects will be now disclosed with reference to the drawings. In the following description, multiple detailed matters will be disclosed to help comprehensive appreciation of one or more aspects. However, it will also be appreciated by one skilled in the art that such aspect(s) can be embodied without the detailed matters. In the following description and the accompanying drawings, specific exemplary aspects of one or more aspects will be described in detail. However, the aspects are exemplary and some of the various methods in principles of various aspects may be used and the descriptions are intended to include all of the aspects and equivalents thereof. Specifically, in "embodiment", "example", "aspect", "illustration" and the like used in the present specification, it may not be construed that any aspect or design that will be described is more favorable or advantageous than other aspects or designs.

Hereinafter, the same reference numerals refer to the same or similar constitutional elements regardless of reference numerals, and overlapping descriptions thereof will be omitted. In addition, in describing an embodiment disclosed in the present specification, a detailed description of related known technologies will be omitted if it is determined that the detailed description makes the gist of the embodiment of the present specification unclear. Further, the accompanying drawings are only for easily understanding the exemplary embodiment disclosed in the present specification and the technical spirit disclosed by the present specification is not limited by the accompanying drawings.

Although the terms "first", "second", and the like are used for describing various devices or constitutional elements, these devices or constitutional elements are not limited by these terms, of course. These terms are merely used for distinguishing one device or constitutional element from another device or constitutional element. Therefore, a first device or constitutional element described below may also be a second device or constitutional element in the technical spirit of the present invention.

Unless otherwise defined, all terms (including technical and scientific terms) used in the present specification may be used as the meaning that may be commonly understood by one skilled in the art to which the present invention belongs. In addition, terms defined in commonly used dictionaries should not be interpreted in an idealized or excessive sense unless defined explicitly and specially.

In addition, the term "or" is intended to mean an inclusive "or" rather than an exclusive "or". That is, unless otherwise specified or clear from the context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in the present specification designates and includes all available combinations of one or more items among enumerated related items.

In addition, the terms "comprise" and/or "comprising" mean that a corresponding feature and/or constitutional element is present but should not be understood as excluding the presence or addition of one or more other features, constitutional elements, and/or groups thereof. Further, unless otherwise specified or in cases where it is not clear from the context to designate a singular form, the singular form in the present specification and claims should be interpreted as meaning "one or more" in general.

When it is mentioned that a certain constitutional element is "connected" or "coupled" to another constitutional element, it should be understood that the certain constitutional element may be directly connected or coupled to the other constitutional element or another intervening constitutional element may be located therebetween. Conversely, when a constitutional element is referred to as being "directly connected" or "directly coupled" to another constitutional element, it is to be understood that there is no intervening constitutional element present.

The suffixes "module" and "unit" or "portion" for constitutional elements used in the following description are given or used interchangeably only for ease of writing the specification, and thus do not themselves have distinct meanings or roles.

The description "a constitutional element or layer is "on" another constitutional element or layer" includes all of cases where the constitutional element or layer is formed directly on the other constitutional element or layer, and where another constitutional element or layer is interposed therebetween. In contrast, the description "a constitutional element is "directly on" another constitutional element refers to a case where there is no intervening constitutional element or layer present.

Spatially relative terms, such as "below", "beneath", "lower", "above" and "upper" may be used herein to easily describe the relationship of one constitutional element to other constitutional element(s) as illustrated in the drawings. It should be understood that the spatially relative terms are intended to encompass different orientations of a device in use or operation, in addition to the orientation depicted in the drawings.

For example, a constitutional element described as "below" or "beneath" another constitutional element could be placed "above" another constitutional element if the constitutional element shown in the drawing is turned over. Thus, the exemplary term "below" or "beneath" may encompass both orientations of "above" and "below" or "beneath". The constitutional element may also be oriented in different directions, and the spatially relative descriptors used herein may be interpreted according to the orientations.

Objects and advantages of the present disclosure and technical elements for accomplishing the objects and advantages will be apparent by referring to embodiments described below in detail in connection with the accompanying drawings. In describing the present disclosure, when the detailed description of the known functions or configurations is determined as unnecessarily obscuring the gist of the present invention, the detailed description will be omitted. In addition, terms described below are defined in consideration of functions in the present disclosure and may be changed depending on a user, the intent of an operator, a custom, or the like.

However, the present disclosure is not limited to the following embodiments and may be implemented in various forms. These embodiments are provided only to make the present disclosure complete and to fully inform one skilled in the art of the scope of the disclosure, and the present disclosure is only defined by the scope of the claims. Therefore, the definition should be made based on the contents throughout the present specification.

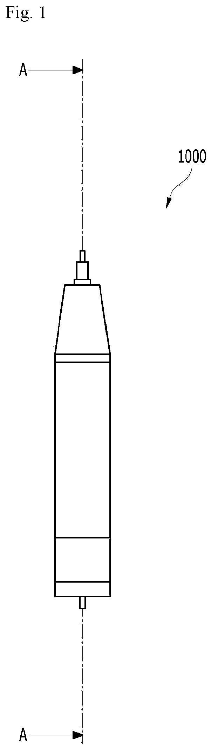

In the present disclosure, an optical coupling apparatus may transmit an optical signal from a static signal path to a rotating signal path. Here, the static signal path may be a path received from an optical coherence tomography (OCT) apparatus, and the rotating signal path may be a path for transmitting a signal to a catheter. Hereinafter, the optical coupling apparatus according to the present disclosure will be described below with reference to FIGS. 1 to 11.

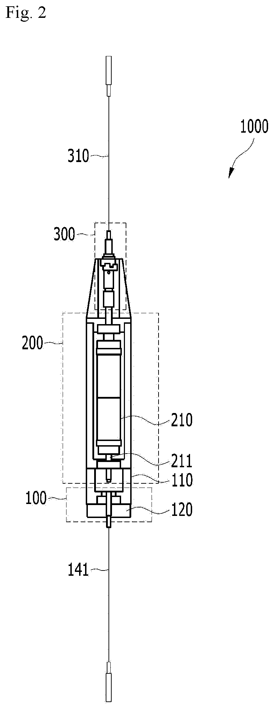

FIG. 1 is a front view for illustrating an example of an' optical coupling apparatus according to some embodiments of the present disclosure. FIG. 2 is a cross-sectional view taken along a line A-A' of FIG. 1 for illustrating the example of the optical coupling apparatus according to some embodiments of the present disclosure.

Referring to FIGS. 1 and 2, an optical coupling apparatus 1000 may include a fixed portion 100, a rotating portion 200 and a connecting portion 300. However, since the above-described constitutional elements are not essential to implement the optical coupling apparatus 1000, the optical coupling apparatus 1000 may include more or fewer constitutional elements than those enumerated above.

The fixing portion 100 may include a first optical path 141 having an optical termination directed in a downstream direction. Here, the downstream direction may be a direction directed toward a downstream rotation optical path (catheter). The first optical path 141 having the optical termination may be a static signal path (non-rotating signal path) transmitted from the optical coherence tomography apparatus.

Here, the first optical path 141 may be a first optical fiber. However, the present disclosure is not limited thereto.

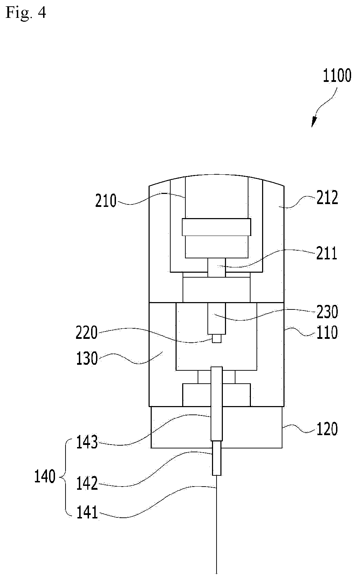

Note that the optical termination of the first optical path 141 may be formed by coupling with a first collimator through fastening with a first ferrule or the like. Here, the ferrule may be a component having a hole in the center in the form of a cylinder tube used for aligning optical fiber core wires in connection with optical fibers. The collimator may be a device that makes a beam of incident light or incident particles parallel. As an example, the collimator may be a ball lens collimator. Hereinafter, the first ferrule and the first collimator will be described with reference to FIG. 4.

The fixing portion 100 may include a fixing portion housing 110 and an aligning plate 120.

The fixing portion housing 110 may form an outer shape of the fixing portion 100. For example, the fixing portion housing 110 may be formed into a cylindrical shape. However, the present disclosure is not limited thereto.

Note that the aligning plate 120 may be coupled to the fixing portion housing 110 to form a lower part of the fixing portion 100.

For example, the aligning plate 120 may be coupled to the fixing portion housing 110 through a bolt or the like.

As another example, the aligning plate 120 may have a male screw thread formed on one region of an outer circumferential surface. In addition, a female screw thread may be formed in one region of the fixing portion housing 110, which corresponds to one region in which the male screw thread is formed, so as to be fastened with the male screw thread. In this case, the fixing portion housing 110 and the aligning plate 120 may be fastened through the screw threads. However, the present disclosure is not limited thereto. For example, a female screw thread may be formed on the outer circumferential surface of the aligning plate 120 and a male screw thread may be formed on the fixing portion housing 110.

Note that, in the present disclosure, the aligning plate 120 may align the first optical path 141 to be located at an accurate position within the optical coupling apparatus 1000.

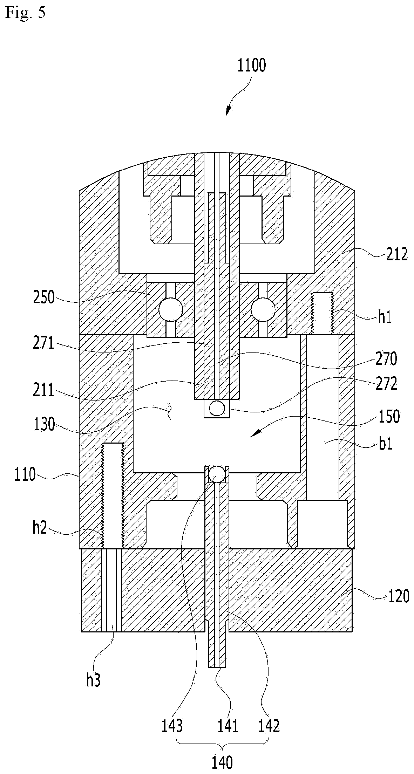

For example, a hole (not shown) through which the first optical path 141 can pass may be provided at the center of the aligning plate 120. The first optical path 141 may be located at the center of the aligning plate 120 through fastening with a ferrule or the like fastened to the hole. At this time, since the aligning plate 120 is fastened to the fixing portion housing 110 through the male screw thread or the like formed on the outer circumferential surface, when the aligning plate 120 is fastened to the fixing portion housing 110, the first optical path 141 may be located at the center of the fixing portion 100. In this case, the first optical path 141 may transmit light to a second optical path described later without loss. However, the present disclosure is not limited thereto. Hereinafter, the fixing portion 100 according to the present disclosure will be described later with reference to FIGS. 4 and 5.

Note that the rotating portion 200 may include a second optical path and have a source of a rotation motion. Here, the second optical path may be a second optical fiber. The second optical fiber is coupled to a rotor of a motor, and may rotate together with the rotor when the rotor rotates. However, the present disclosure is not limited thereto. Hereinafter, the second optical path will be described later with reference to FIGS. 4 and 5.

Note that, in the present disclosure, the second optical path may include a first end portion (not shown) and a second termination (not shown).

The first end portion may be directed in a direction where the optical termination of the first optical path 141 is located. However, the present disclosure is not limited thereto.

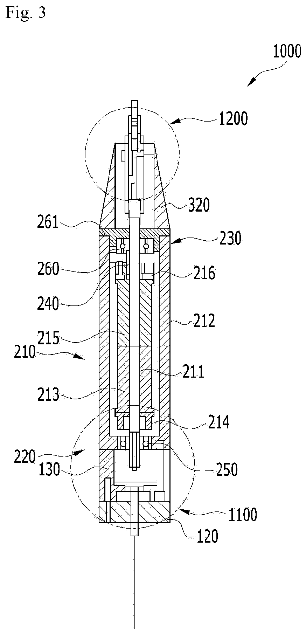

Note that the second termination may be directed in a direction where a downstream rotation optical path is located. However, the present disclosure is not limited thereto. Hereinafter, the first end portion and the second termination will be described with reference to FIG. 3.

Note that the source of the rotation motion may be a motor 210 or the like that allows the rotating portion 200 to rotate. However, the present disclosure is not limited thereto.

Specifically, the rotating portion 200 may include a motor 210. The rotating portion 200 may be rotated by the motor 210. However, the present disclosure is not limited thereto.

Note that the motor 210 may include a rotor 211. In this case, the rotor 211 may be a hollow shaft. In this case, the second optical path (not shown) may be inserted into the hollow shaft. However, the present disclosure is not limited thereto. Hereinafter, the motor will be described later with reference to FIGS. 4 and 5.

Note that the connecting portion 300 may provide a connection between the rotating portion 200 and a downstream rotation optical path 310. Here, the downstream rotation optical path 310 may be an optical fiber.

Specifically, the connecting portion 300 may provide a connection so that the downstream rotation optical path 310 can be rotatably coupled to the rotating portion 200.

For example, the downstream rotation optical path 310 may be rotatably coupled to the rotating portion 200 through a fiber connector or the like, which will be described later. In this case, the downstream rotation optical path 310 may rotate together with rotation of the rotating portion 200. However, the present disclosure is not limited thereto.

Note that, according to some embodiments of the present disclosure, the downstream rotation optical path 310 may be provided to be attachable or detachable to or from the connecting portion 300. However, the present disclosure is not limited thereto.

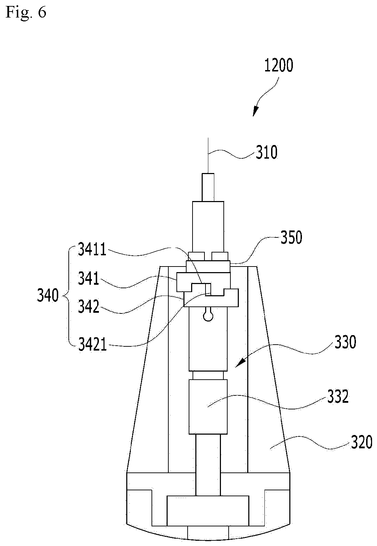

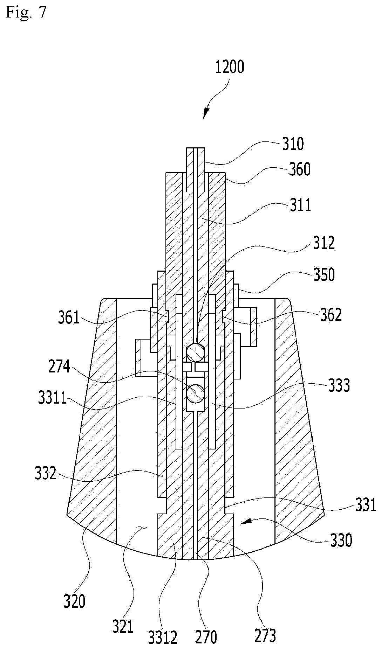

Note that, according to some embodiments of the present disclosure, the connecting portion 300 may further include a pair of counterweights that increases clamping force of the connection between the rotating portion 200 and the downstream rotation optical path 310 as a rotation speed of the rotating portion 200 increases. Hereinafter, the pair of counterweights according to the present disclosure will be described later with reference to FIGS. 6 and 7.

According to the configuration described above, the optical coupling apparatus 1000 may transmit an optical signal from a static signal path to a rotating signal path.

FIG. 3 is a detailed view of FIG. 2 for illustrating the example of the optical coupling apparatus according to some embodiments of the present disclosure.

Referring to FIG. 3, the optical coupling apparatus 1000 may include a first side surface 1100, a second side surface 1200, the fixed portion 100, the rotating portion 200 and the connecting portion 300. However, the present disclosure is not limited thereto.

The first side surface 1100 can provide first optical bonding. Here, the first optical bonding may be optical bonding between the first optical path 141 and the second optical path.

Specifically, the first optical path 141 may be a fixed optical path, and the second optical path may be a rotation optical path. The first optical path 141 and the second optical path may be spaced apart from each other by a predetermined distance. In this case, the first optical path 141 may be terminated by a first collimator (not shown), and one end of the second optical path may be terminated by a second collimator (not shown). Here, the collimator may be a device that makes a beam of incident light or incident particles parallel. In this case, light incident through the first optical path 141 may be transferred to the second optical path through the first collimator and the second collimator. However, the present disclosure is not limited thereto. Hereinafter, the first side surface 1100 will be described later with reference to FIGS. 4 and 5.

Note that the second side surface 1200 may provide second optical bonding.

Specifically, the second side surface 1200 may provide second optical bonding between the second optical path and the downstream rotation optical path.

More specifically, the second optical path and the downstream rotation optical path may be rotation optical paths. The second optical path and the downstream rotation optical path may be spaced apart from each other by a predetermined distance. In this case, the other end opposite to the one end of the second optical path may be terminated by a third collimator (not shown), and the downstream rotation optical path may be terminated by a fourth collimator (not shown). In this case, the light incident on the second optical path may be transferred to the downstream rotation optical path through the third collimator and the fourth collimator. However, the present disclosure is not limited thereto. Hereinafter, the second side surface 1200 will be described later with reference to FIGS. 6 and 7.

Note that the fixing portion 100 may be coupled to the lower part of the rotating portion 200.

For example, the fixing portion 100 may be coupled to the rotating portion 200 through a bolt or the like.

As another example, the fixing portion 100 may have a male screw thread formed on one region of the outer circumferential surface. In addition, a female screw thread may be formed in one region of the rotating portion 200, which corresponds to one region in which the male screw thread is formed, so as to be fastened with the male screw thread. In this case, the fixing portion 100 and the rotating portion 200 may be fastened through the screw threads. However, the present disclosure is not limited thereto. For example, a female screw thread may be formed on the outer circumferential surface of the fixing portion 100 and a male screw thread may be formed on the rotating portion 200. Hereinafter, a coupling relationship between the fixing portion 100 and the rotating portion 200 will be described later with reference to FIG. 5.

Note that the rotating portion 200 may include a motor 210, a first end portion 220, a second termination 230, an aligning spring 240, a first bearing 250 and a second bearing 260. However, the present disclosure is not limited thereto.

In the present disclosure, the motor 210 may be an AC motor, a DC motor, a brushless DC (BLDC) motor, a servo motor, a linear motor, or the like. However, the present disclosure is not limited thereto, and various motors may be used as the motor 210 according to some embodiments of the present disclosure.

Note that the motor 210 may include a rotor 211, a motor housing 212, a first magnetic cylinder 213, a first rotor support 214, a second magnetic cylinder 215, a second rotor support 216 and a motor bore (not shown). However, the present disclosure is not limited thereto.

The motor bore may be a space for allowing the rotor 211, the motor housing 212, the first magnetic cylinder 213, the first rotor support 214, the second magnetic cylinder 215 and the second rotor support 216 to be provided inside the motor 210. However, the present disclosure is not limited thereto.

Note that the rotor 211 may be a shaft that rotates through the first magnetic cylinder 213 and the second magnetic cylinder 215. In this case, the rotor 211 may be a hollow shaft. In this case, the second optical path may be inserted into the rotor 211, and may rotate together with the rotor 211 when the rotor 211 rotates. However, the present disclosure is not limited thereto.

Note that the motor housing 212 may form an outer shape of the motor 210. However, the present disclosure is not limited thereto, and the motor housing 212 may form an outer shape of the rotating portion 200.

Note that the first magnetic cylinder 213 and the second magnetic cylinder 215 may rotate inside the motor 210. Here, the first magnetic cylinder 213 and the second magnetic cylinder 215 may generate magnetism and rotate according to power supplied from a power supply unit (not shown). In this case, the first magnetic cylinder 213 and the second magnetic cylinder 215 may rotate the rotor 211. However, the present disclosure is not limited thereto.

Note that the first magnetic cylinder 213 may be coupled to the rotor 211 through the first rotor support 214. The second magnetic cylinder 215 may be coupled to the rotor 211 through the second rotor support 216. In this case, the rotor 211 may be coupled to a first bearing and a second bearing described later. Therefore, the first magnetic cylinder 213 and the second magnetic cylinder 215 may rotate together with the rotation of the rotor 211. However, the present disclosure is not limited thereto.

Note that the first end portion 220 may be directed in a direction where the optical termination of the first optical path 141 is located. Here, the direction where the optical termination of the first optical path 141 is located may be a direction where the fixing portion 100 is provided. That is, the first end portion 220 may be a region where the rotating portion 200 and the fixing portion 100 are fastened. However, the present disclosure is not limited thereto.

Note that the second termination 230 may be directed in a direction where the downstream rotation optical path 310 is located. Here, the direction where the downstream rotation optical path 310 is located may be a direction where the connecting portion 300 is provided. That is, the second termination 230 may be a region where the rotating portion 200 and the connecting portion 300 are fastened. However, the present disclosure is not limited thereto.

Note that one region of each of the first end portion 220 and the second termination 230 may be provided with a first bearing 250 and a second bearing 260 allowing the rotor 211 of the motor 210 to rotate.

Specifically, the first bearing 250 may be provided in one region of the first end portion 220 of the rotating portion 200. The second bearing 260 may be provided in one region of the second termination 230 of the rotating portion 200. In this case, the rotor 211 of the motor 210 may be combined with the first bearing 250 and the second bearing 260 to rotate. However, the present disclosure is not limited thereto.

Note that, in the present disclosure, the second bearing 260 may be prevented from interfering with the connecting portion 300 by a bearing cap 261.

Specifically, the bearing cap 261 may be coupled to an upper side of the first bearing 250. The rotating portion 200 may be coupled to the bearing cap 261. However, the present disclosure is not limited thereto.

Note that the aligning spring 240 may be provided to surround the rotor 211 of the motor 210 and may be disposed between the second bearing 260 and the second rotor support 216. In this case, the aligning spring 240 may align the rotor 211 so that the rotor 211 is located at the center of the optical coupling apparatus 1000. However, the present disclosure is not limited thereto.

FIG. 4 is a view for illustrating an example of a first aspect according to some embodiments of the present disclosure. FIG. 5 is a detailed view of FIG. 4 for illustrating the example of the first aspect according to some embodiments of the present disclosure.

Referring to FIGS. 4 and 5, the first side surface 1100 may provide a first optical bonding 150. Here, the first optical bonding 150 may be an optical bonding between a fixed light source 140 and a second optical path 270.

Specifically, the fixed light source 140 may include a first optical path 141. Here, the first optical path 141 may be a path for receiving an optical signal from the optical coherence tomography apparatus. The second optical path 270 may be located at a position spaced apart from the termination of the first optical path 141 by a predetermined distance. In this case, the light transmitted by the first optical path 141, which is a static signal path, may be transferred to the second optical path 270, which is a rotating signal path. However, the present disclosure is not limited thereto.

Note that the fixed light source 140 may include the first optical path 141, a first ferrule 142, and a first collimator 143. The first optical path 141 may be coupled to the first collimator 143 through fastening with the first ferrule 142. In this case, the first optical path 141 may transmit an optical signal to the second optical path 270 through the first collimator 143. However, the present disclosure is not limited thereto.

Note that the second optical path 270 may be inserted into the rotor 211 and rotate together with the rotor 211 as the rotor 211 rotates. In this case, the rotor 211 may be a hollow shaft. In this case, the second optical path 270 may be inserted into the hollow shaft. The second optical path 270 may receive light from the first optical path 141 through the second collimator 272.

Specifically, the second optical path 270 may be coupled to the second collimator 272 through fastening with a second ferrule 271. In this case, the second optical path 270 may receive an optical signal from the first optical path 141 through the second collimator 272. However, the present disclosure is not limited thereto.

Note that, in the present disclosure, the fixing portion 100 may include the first bore 130 in which the constitutional elements described above may be provided.

For example, at least a portion of the fixed light source 140, at least a portion of the rotor 211, and the like may be provided in the first bore 130. However, the present disclosure is not limited thereto.

Note that, in the present disclosure, the first bearing 250 may be provided in one region of the first end portion 220 of the rotating portion 200.

Specifically, the first bearing 250 may be provided at one end of the motor housing 212. In this case, the rotor 211 is fastened with the first bearing 250, and therefore, can rotate when power is generated from the first magnetic cylinder 213 and the second magnetic cylinder 215. However, the present disclosure is not limited thereto.

Note that the fixing portion 100 and the rotating portion 200 may be coupled through at least one bolt or the like.

Specifically, the motor housing 212 may be provided with at least one first fastening hole h1. At least one first bolt b 1 may be provided in one region of the fixing portion 100 corresponding to at least one first fastening hole h1. In this case, the fixing portion 100 and the rotating portion 200 may be coupled through fastening of at least one first bolt b1 and at least one first fastening hole h1. However, the present disclosure is not limited thereto.

Note that, in the present disclosure, the fixing portion 100 may include at least one bolt groove into which at least one first bolt b1 is inserted. In this case, at least one first bolt b 1 may be inserted into at least one bolt groove and fastened to at least one first fastening hole h1. However, the present disclosure is not limited thereto.

Note that the fixing portion 100 and the aligning plate 120 may be coupled through at least one bolt or the like.

Specifically, the fixing portion 100 may be provided with at least one second fastening hole h2. At least one third fastening hole h3 may be provided in one region of the aligning plate 120 corresponding to at least one second fastening hole h2. In this case, the fixing portion 100 and the aligning plate 120 may be coupled through at least one bolt passing through at least one third fastening hole h3 and fastened to at least one second fastening hole h2.

FIG. 6 is a view for illustrating an example of a second aspect according to some embodiments of the present disclosure. FIG. 7 is a detailed view of FIG. 6 for illustrating the example of the second aspect according to some embodiments of the present disclosure.

Referring to FIGS. 6 and 7, the connecting portion 300 may be located on the second side surface 1200.

In this case, the second side surface 1200 may include a downstream rotation optical path 310, a cap 320, a fiber connector 330, a pair of counterweights 340, an elastic member 350 and a coil 360. However, the present disclosure is not limited thereto.

The cap 320 may form an outer shape of the second side surface 1200 and protect at least one constitutional element included in the connecting portion 300. One end of the cap 320 may be coupled and fixed to the rotating portion 200.

For example, the cap 320 may be coupled to the rotating portion 200 through an adhesive or the like. However, the present disclosure is not limited thereto.

Note that the cap 320 may include a second bore 321. In addition, the constitutional elements such as the connecting portion 300 and the pair of counterweights 340 may be provided in the second bore 321. However, the present disclosure is not limited thereto.

Note that the fiber connector 330 may be provided inside the cap 320 to provide a second optical bonding between the downstream rotation optical path 310 and the second optical path 270 inserted into the rotor 211.

Specifically, the fiber connector 330 may include the second optical path 270 inserted into the rotor 211 and coupled to the fiber connector 330 and the downstream rotation optical path 310 coupled by a pair of fingers (not shown).
In this case, the second optical path 270 may be coupled to the third collimator 274 through fastening with a third ferrule 273. The downstream rotation optical path 310 coupled to a fourth collimator 312 through fastening with a fourth ferrule 311 may be located at a position spaced apart from the termination of the second optical path 270 by a predetermined distance. In this case, an optical signal received by the second optical path 270 from the first optical path 141 may be transferred to the downstream rotation optical path 310 through the third collimator 274 and the fourth collimator 312. However, the present disclosure is not limited thereto.

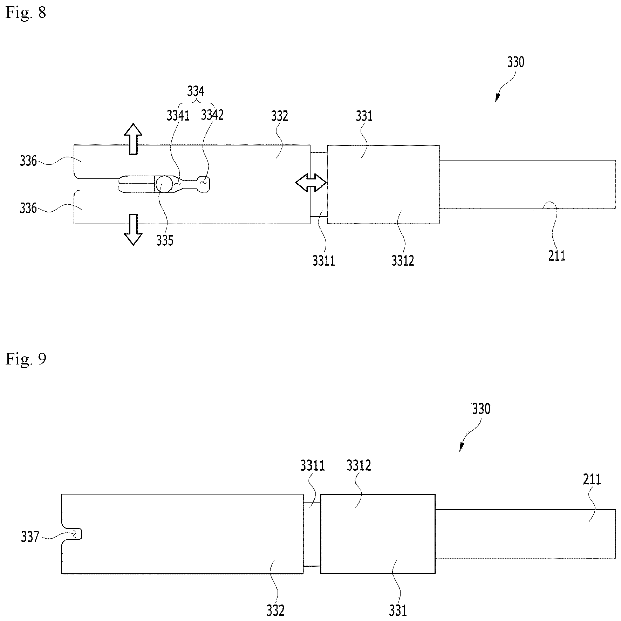

Note that the fiber connector 330 may include an aligning sleeve 333. Here, the aligning sleeve 333 may align axes of the third collimator 274 and the fourth collimator 312 each other. However, the present disclosure is not limited thereto. Hereinafter, the fiber connector 330 will be described with reference to FIGS. 8 and 9.

Note that the pair of counterweights 340 may increase clamping force of the connection between the rotating portion 200 and the downstream rotation optical path 310 as a rotation speed of the rotating portion 200 increases.

Specifically, the pair of counterweights 340 may include a first counterweight 341 and a second counterweight 342. In this case, the first counterweight 341 and the second counterweight 342 may be engaged with each other so that central axes thereof are not matched and rotation axes thereof are matched. In this case, the pair of counterweights 340 may increase the clamping force between the rotating portion 200 and the downstream rotating optical path 310 by centripetal force (or centrifugal force) generated by rotating together as the rotor 211 rotates. Further, in this case, the pair of counterweights 340 may increase the clamping force between the fiber connector 330 and the downstream rotation optical path 310. However, the present disclosure is not limited thereto.

Note that, according to some embodiments of the present disclosure, the first counterweight 341 may include at least one first notch 3411 formed along a circumference. Here, at least one notch 3411 may be a groove or the like recessed along the circumference. The second counterweight 342 may also include at least one second notch 3421 formed along a circumference. At least one first notch 3411 of the first counterweight 341 and at least one second notch 3421 of the second counterweight 342 may be provided to face each other and coupled.

Note that, according to some embodiments of the present disclosure, at least one first notch 3411 and at least one second notch 3421 may be provided to cross each other. In this case, the first counterweight 341 and the second counterweight 342 may be coupled by at least one notch and a protrusion formed by the at least one notch.

For example, the first counterweight 341 may have a protrusion formed by at least two first notches 3411. In this case, the protrusion of the first counterweight 341 may be inserted into at least one second notch 3421 of the second counterweight 342. However, the present disclosure is not limited thereto.

Note that the elastic member 350 may maintain the connection between the rotating portion 200 and the downstream rotation optical path 310 when the rotating portion 200 does not rotate.

For example, the elastic member 350 may be a rubber band or rubber string.

Therefore, the connection between the rotating portion 200 and the downstream rotation optical path 310 may be made by the pair of counterweights 340 when the rotating portion 200 rotates, and may be maintained by the elastic member 350 when the rotating portion 200 does not rotate. However, the present disclosure is not limited thereto.

Note that, according to some embodiments of the present disclosure, the elastic member 350 may deflect a pair of fingers (not shown) provided to the fiber connector 330 toward each other, thereby enabling the pair of fingers to fix the downstream rotation optical path (310). However, the present disclosure is not limited thereto. Hereinafter, the fiber connector 330 will be described later with reference to FIGS. 8 and 9.

Note that the coil 360 may be formed to surround the fourth ferrule 311 and protect the fourth ferrule 311. The coil 360 may be coupled to the fourth ferrule 311 and the fiber connector 330 through a first coupling 361 and a second coupling 362. However, the present disclosure is not limited thereto.

Note that the coil 360 may be formed to surround a catheter (not shown). The coil 360 may protect the downstream rotation optical path 310 provided in the catheter. However, the present disclosure is not limited thereto.

FIG. 8 is a front view for illustrating an example of a fiber connector according to some embodiments of the present disclosure. FIG. 9 is a plan view for illustrating the example of the fiber connector according to some embodiments of the present disclosure.

Referring to FIGS. 8 and 9, the fiber connector 330 may include a fiber connector body portion 331, a sleeve 332, a slot 334, a pin 335, a pair of fingers 336 and a groove 337. However, the present disclosure is not limited thereto.

The fiber connector body portion 331 may form an outer shape of the fiber connector 330. For example, the fiber connector body portion 331 may be formed into a hollow pipe shape. In this case, a portion of the rotor 211 of the motor 210 may be inserted into the fiber connector body portion 331. However, the present disclosure is not limited thereto.

Note that the fiber connector body portion 331 may be formed with a first region 3311 and a second region 3322. Here, the first region 3311 may have a diameter smaller than that of the second region 3322 and may be a region where the sleeve 332 is coupled to an outer circumferential surface. The second region 3322 may be a region into which at least a portion of the rotor 211 is inserted. However, the present disclosure is not limited thereto.

Note that the sleeve 332 may be movably provided in one region of the fiber connector body portion 331. The sleeve 332 may provide a connection between the fiber connector 330 and the downstream rotation optical path 310 or a connection between the fiber connector 330 and the catheter.

Specifically, the sleeve 332 may include the slot 334, the pin 335 and the pair of fingers 336. In this case, the pair of fingers 336 may fix or separate the downstream rotation optical path 310, depending on a position where the sleeve 332 is located.

More specifically, the slot 334 may be provided in one region of the outer circumferential surface of the sleeve 332. The pin 335 may be inserted into the slot 334. In this case, as the sleeve 332 moves to the left or right, a position of the slot 334 in which the pin 335 is located may be changed.

For example, when the sleeve 332 is pulled to the left, the pin 335 may be located at a second position 3342 of the slot 334. In this case, the pair of fingers 336 may separate the downstream rotation optical path 310. When the sleeve 332 is moved to the right, the pin 335 may be located at a first position 3341 of the slot 334. In this case, the pair of fingers 336 may fix the downstream rotation optical path 310. However, the present disclosure is not limited thereto. For example, the pair of fingers 336 may separate the downstream rotation optical path 310 when the pin 335 is located at the first position 3341, and fix the downstream rotation optical path 310 when the pin 335 is located at the second position 3342.

Note that the groove 337 may prevent the catheter and the fiber connector 330 from rotating separately without rotating together. For example, the catheter may be provided with a fastening portion that is fastened to the groove 337. In this case, the catheter and the fiber connector 330 may rotate together through the groove 337 and the fastening portion. However, the present disclosure is not limited thereto.

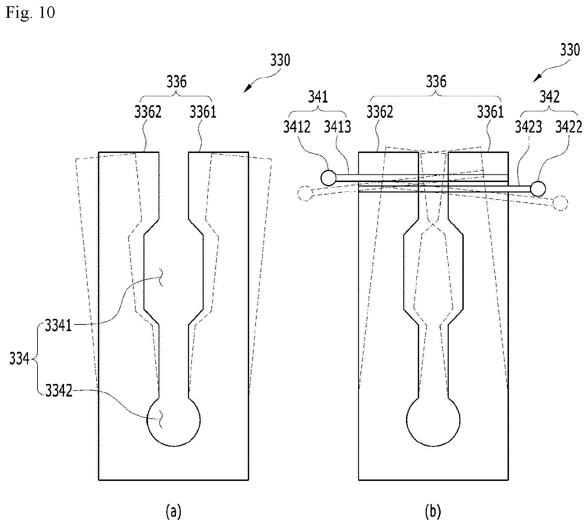

FIG. 10 is a view for illustrating an example of a pair of counterweights according to some embodiments of the present disclosure.

Referring to FIG. 10(a), the fiber connector 330 may include the pair of fingers 336. The pair of fingers 336 may fix a downstream rotation optical path (not shown) or separate the downstream rotation optical path according to a position of the slot 334 in which a pin (not shown) is located.

In this case, as the rotating portion 200 rotates, each of the pair of fingers 336 may be opened by centrifugal force. In this case, a problem may occur in that the pair of fingers 336 cannot fix the downstream rotation optical path. Therefore, according to some embodiments of the present disclosure, each of the pair of counterweights 340 may be coupled to each of the pair of fingers 336.

Specifically, referring to FIG. 10(b), the first counterweight 341 may include a first coupling portion 3413 and a first weight portion 3412. Here, the first weight portion 3412 may be provided at one end of the first coupling portion 3413, and the other end of the first coupling portion 3413 may be coupled to the first finger 3361. In this case, the first coupling portion 3413 may not be coupled to the second finger 3362.

More specifically, when the other end of the first coupling portion 3413 is coupled to the first finger 3361, the first weight portion 3412 provided at one end of the first coupling portion 3413 may be present in a direction where the second finger 3362 is located. In this case, the weight may be concentrated on the first weight portion 3412 of the first counterweight 341. Therefore, the centrifugal force of the first weight portion 3412 may be generated in an opposite direction to the centrifugal force of the first finger 3361 generated as the rotating portion 200 rotates. The first weight portion 3412 may have a preset weight so as to generate centrifugal force greater than a magnitude of the centrifugal force of the first finger 3361.

In addition, the second counterweight 342 may include a second coupling portion 3423 and a second weight portion 3422. Here, the second weight portion 3422 may be provided at one end of the second coupling portion 3423, and the other end of the second coupling portion 3423 may be coupled to the second finger 3362. In this case, the second coupling portion 3423 may not be coupled to the first finger 3361.

More specifically, when the other end of the second coupling portion 3423 is coupled to the second finger 3362, the second weight portion 3422 provided at one end of the second coupling portion 3423 may be present in a direction where the first finger 3361 is located. In this case, the weight may be concentrated on the second weight portion 3422 of the second counterweight 342. Therefore, the centrifugal force of the second weight portion 3422 may be generated in an opposite direction to the centrifugal force of the second finger 3362 generated as the rotating portion 200 rotates. The second weight portion 3422 may have a preset weight so as to generate centrifugal force greater than a magnitude of the centrifugal force of the second finger 3362.

That is, when the rotating portion 200 rotates, the respective centrifugal forces of the pair of counterweights (first counterweight 341 and second counterweight 342) may be generated in a direction to cancel the respective centrifugal forces of the first finger 3361 and the second finger 3362. Here, the centrifugal force of each of the pair of counterweights may be greater than the centrifugal force of each of the first finger 3361 and the second finger 3362. In this case, the clamping force of the pair of fingers 336 fixing the downstream rotation optical path may be increased through the pair of counterweights 340.

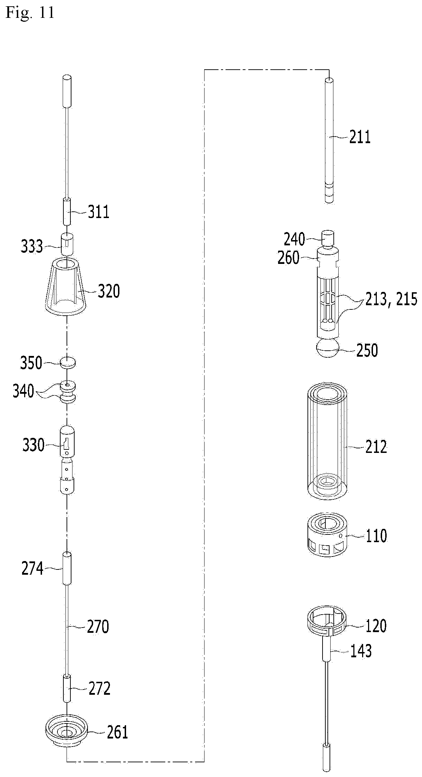

FIG. 11 is an exploded perspective view for illustrating an example of an optical coupling apparatus according to some embodiments of the present disclosure.

Referring to FIG. 11, the optical coupling apparatus 1000 may include the fixed portion 100, the rotating portion 200 and the connecting portion 300.

The connecting portion 300 may include the fiber connector 330, the pair of counterweights 340, and the elastic member 350. The connecting portion 300 may fix the fourth ferrule 311 to the optical coupling apparatus 1000 through the fiber connector 330. In this case, the connecting portion 300 may be prevented from being directly exposed to the outside through the cap 320. However, the present disclosure is not limited thereto.

Note that the rotating portion 200 may include the second optical path 270 through the rotor 211 provided inside the motor 210.

Specifically, the rotor 211 may be a pipe-shaped hollow shaft. In this case, the second optical path 270 may be inserted into the rotator 211, and therefore, may rotate together with the rotor 211 as the rotor 211 is rotated by the first magnetic cylinder 213 and the second magnetic cylinder 215. However, the present disclosure is not limited thereto.

Note that the second optical path 270 may include the second collimator 272 for receiving an optical signal from the first optical path 141 and the third collimator 274 for transmitting the optical signal to the downstream rotating optical path 310. However, the present disclosure is not limited thereto.

Note that the fixing portion housing 110 may be coupled to the motor housing 212 of the rotating portion 200 through at least one bolt or the like. In this case, the aligning plate 120 for aligning the first optical path 141 may be coupled to a lower end of the fixing portion housing 110. However, the present disclosure is not limited thereto.

Note that, according to some embodiments of the present disclosure, in a method for transmitting an optical signal from a static signal path to a rotating signal path in the optical coupling apparatus 1000, the downstream rotation optical path 310 may be coupled to one end of the optical coupling apparatus 1000.

Specifically, in the optical coupling apparatus 1000, the downstream rotation optical path 310 may be coupled through the connecting portion 300.

More specifically, in the optical coupling apparatus 1000, the downstream rotation optical path 310 may be coupled through the fiber connector 330 provided to the connecting portion 300. However, the present disclosure is not limited thereto.

Note that when the downstream rotating optical path 310 is coupled, the optical coupling apparatus 1000 may receive an optical signal from the first optical path 141 included in the fixing portion 100 and having an optical termination directed in a downstream direction. That is, the optical coupling apparatus 1000 may receive an optical signal from the optical coherence tomography apparatus through the first optical path 141. However, the present disclosure is not limited thereto.

Note that the optical coupling apparatus 1000 may transfer the optical signal received from the first optical path 141 to the second optical path 270 provided in the rotating portion 200 through the first optical bonding. The optical coupling apparatus 1000 may transfer the optical signal transmitted to the second optical path 270 to the downstream rotation optical path 310 through the second optical bonding.

In this case, the optical coupling apparatus 1000 may rotate the rotating portion 200 and the downstream rotation optical path 310 together through the connecting portion 300 providing a connection between the rotating portion 200 and the downstream rotation optical path 310. However, the present disclosure is not limited thereto.

The description of the presented embodiments has been provided to allow anyone skilled in the art to use or embody the present disclosure. It will be apparent to one skilled in the art that various modifications may be made to the embodiments, and general principles defined herein may be applied to other embodiments without departing from the scope of the present disclosure. Therefore, the present disclosure is not limited to the embodiments presented herein and should be interpreted as having the broadest possible range that is consistent with the principles and novel features presented herein.

## Claims

1. An apparatus for transmitting an optical signal from a static signal path to a rotating signal path comprising:
a fixing portion comprising a first optical path having an optical termination directed in a downstream direction;
a rotating portion comprising a second optical path and having a source of a rotation motion, wherein the second optical path comprises a first end portion directed in a direction where the optical termination is located and a second end portion directed in a direction where a downstream rotation optical path is located; and
a connecting portion providing a connection between the rotating portion and the downstream rotation optical path.

2. The apparatus of claim 1, wherein the connecting portion comprises a pair of counterweights which increases clamping force of the connection between the rotating portion and the downstream rotation optical path as a rotation speed of the rotating portion increases.

3. The apparatus of claim 2, wherein the pair of counterweights comprises at least one notch formed along a circumference and notches of the pair of counterweights are provided to face each other.

4. The apparatus of claim 2, wherein the pair of counterweights is engaged with each other so that central axes of the counterweights are not matched and rotation axes of the counterweights are matched.

5. The apparatus of claim 1, wherein the connecting portion comprises an elastic member which maintains the connection between the rotating portion and the downstream rotation optical path, when the rotating portion does not rotate.

6. The apparatus of claim 1, wherein the second optical path is an optical fiber configured to rotate together with the rotation portion together when the rotation portion rotates.

7. The apparatus of claim 6, wherein the source of the rotation motion is a motor, and the optical fiber rotates while being coupled to a rotor of the motor.

8. The apparatus of claim 7, wherein the rotor comprises a hollow shaft to which the optical fiber is inserted.

9. The apparatus of claim 1, wherein the downstream rotation optical path is provided to be attachable to the connecting portion or to be detachable from the connecting portion.

10. The apparatus of claim 1, wherein the connecting portion comprises a fiber connector which fixes the downstream rotation optical path, wherein the fiber connector comprises:
a slot provided on an outer circumferential surface of the fiber connector;
a pin which is movable while being inserted to the slot; and
a pair of fingers which separates the downstream rotation optical path or fixes the downstream rotation optical path according to a position of the slot in which the pin is located.

11. A method for transmitting an optical signal from a static signal path to a rotating signal path comprising:
coupling an apparatus and a downstream rotation optical path;
receiving an optical signal from a first optical path having an optical termination directed in a downstream direction, wherein the first optical path is included in a fixing portion of the apparatus; and
rotating a rotating portion of the apparatus and the downstream rotation optical path together through a connecting portion of the apparatus, which provides a connection between the rotating portion and the downstream rotation optical path.
